# EUROPEAN PATENT SPECIFICATION

(11) **EP 0 293 080 B1**
(45) Date of publication and mention of the grant of the patent: **29.06.1994**
(21) Application number: 88303633.7
(22) Date of filing: 21.04.1988
(51) Int. Cl.: A61N 5/02

(54) **Electronic treatment device using microwaves**
Elektronisches Therapiegerät für Mikrowellen
Dispositif pour traitement électronique utilisant des micro-ondes

(30) Priority: 28.05.1987 JP 82103/87 U; 19.01.1988 JP 9253/88
(43) Date of publication of application: 30.11.1988
(73) Proprietor: Takase, Haruo, Tokyo (JP)
(72) Inventor: Takase, Haruo, Tokyo (JP)
(74) Representative: Ben-Nathan, Laurence Albert

(56) References cited:
- DE-A- 2 701 934
- FR-E- 58 199
- US-A- 4 316 474
- PROCEEDINGS OF A SPECIAL SYMPOSIUM ON CRITICAL EMERGING ISSUES IN BIOMEDICAL ENGINEERING, 1986, p.76-78, IEEE, New York, US; J.W. STROHBEHN: "Use of heat in cancer therapy"

## Description

The present invention relates to an electronic treatment device using microwaves, and more particularly it relates to a treatment applying microwaves of less than 6 to 15 m to a lesion of a patient by locally heating the lesion to a predetermined temperature within a certain temperature range.

As microwaves travel linearly, they can be focused in a one-directional beam, and effectively heat the human body to the depth of skeletal tissues even when the patient is clothed, applications of microwaves to the living body are being studied increasingly.

Such application, however, is still at the stage of research, and the practical application is limited to therapy of stiff shoulders and lumbagoes by accelerating circulation and improving metabolism by utilizing its heating effect.

Subsequent researches on microwaves established a sure and accurate control of local heating by controlling the intensity and direction of super microwaves of 12.24 cm to thereby cause them to reach the tissues within the living body.

The inventor of the present invention has successfully developed a practical therapeutic device and filed Japanese Utility Model Application UM 62-82103 from which the present application claims priority. The device comprises a bed and a protective frame placed on the bed along the longitudinal direction thereof having a cross section of an inverted U shape, and is characterized in that a microwave irradiation means is incorporated on the upper side of the protective frame and the lower side of the bed along the longitudinal direction of the bed in a freely reciprocating fashion to irradiate microwaves toward the bed.

This therapeutic device enables irradiating the microwaves to the lesion inside the patient body by precisely controlling the direction and intensity, and heating the lesion to a prescribed therapeutic temperature.

However, the therapeutic effects are not achieved uniformly since the temperature regarded as effective varies depending on the types of the disease such as malignant tumors, the size of the lesion, or the types of viruses. It is further problematic in that the temperature at which optimum therapeutic effect can be achieved is not established for destroying malignant cellular tissues or the mechanism between death of various viruses and damage to the living body is not clearly elucidated.

DE-A-2,701,934 illustrates a treatment device having the features of the preamble of Claim 1.

The primary object of the present invention is to provide an improved electronic treatment device which makes it possible to achieve an optimum and balanced therapeutic effect based on the result of theoretical and clinical researches on diseases such as malignant tumors or destruction of various viruses and effect on the living body.

The present invention also makes it possible to provide a microwave irradiation means which can freely set the heating temperature within a predetermined range for irradiation of microwaves to lesions.

Features of the present invention may enable fine adjustment by the patient who is being treated by arbitrarily controlling the heating means for the surface temperature.

Further features of the present invention may enable arbitrary control of irradiation time of the microwaves.

The invention provides an electronic treatment device in accordance with Claim 1.

In accordance with the present invention, the lesions of a patient are treated by movable microwave irradiating means which are incorporated in the device at a temperature in the range at 37 - 70°C.

In the drawings:-
Fig. 1 is a perspective view of the electronic treatment device illustrating one embodiment of the present invention;
Fig. 2 is a cross sectional view seen along the arrow at line A-A in Fig. 1; and

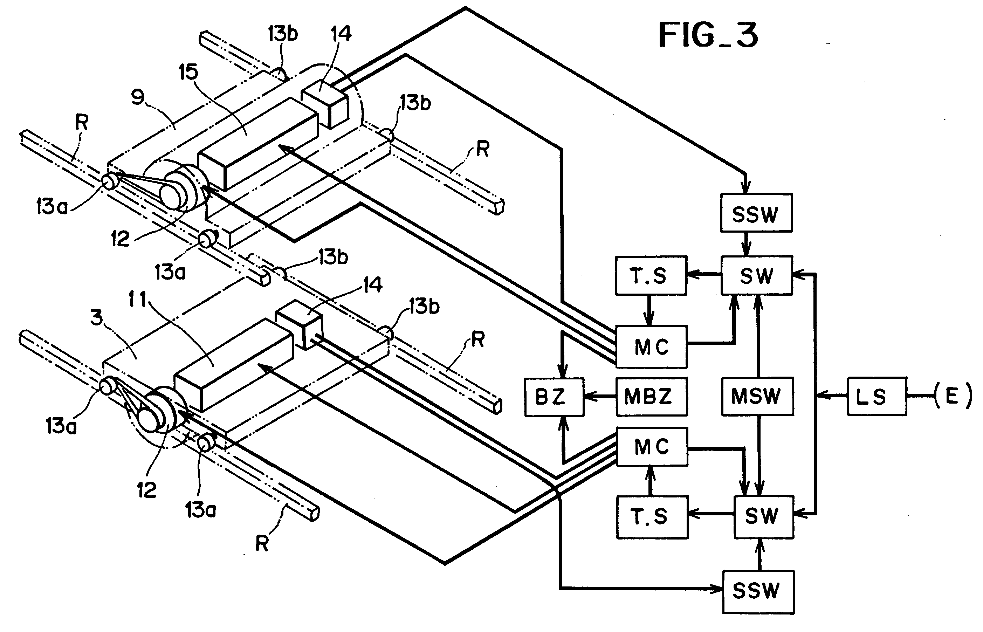

Fig. 3 is a diagrammatic perspective view of the microwave irradiating tubes of the device of Figs. 1 and 2 and control means therefor.

The present invention offers an efficient therapeutic means utilizing microwave irradiation to heat the body of a patient or his lesion and eliminates the need for surgical operation, and the details of the invention are explained concretely.

The microwaves heat the body of a patient or the lesion in his/her body gradually to a predetermined temperature within the range of 37 - 70°C, and malignant tissues or viruses are gradually destroyed and disappear after the predetermined temperature is reached, and heal the patient without damaging the body too seriously.

As for the optimum temperature of heating the lesion by microwave irradiation, it is not necessarily constant as it depends on the size of the lesion, the types of malignant tumors or other diseases, the types and character of viruses, and the conditions of a patient. However, it should be above 37°C since it is not effective if below the natural body temperature (36.5°C). If it is above 70°C, it may destory the living tissues and therefore the upper limit should be set at 70°C.

Thus, the temperature range of 37 - 70°C has been arrived at through experience as well as various experiments as the most preferred range for destroying malignant tumors and other malignant cellular tissues, or treating various viral diseases.

In order to perform rapid treatment within the temperature range of 37 - 70°C by microwaves, there is provided a means to adjust the temperature for every 1°C if below 40°C, and for every 0.5°C if above 40°C in order to destroy the malignant cellular tissues and to minimize damages to the living tissues, or to destory viruses.

It will also be useful to provide a means whereby a patient may make fine adjustment for said temperature adjustment within the above mentioned range so that he may freely control the heating temperature to that which he feels warm enough.

In order to ensure the safety of the microwave thermal therapy and to assure the patient of his safety, an alarm means to emit voiced or optical alarms at the predetermined levels such as at 40.5°C, 55.5°C and 60.5°C, or a thermostat means to automatically suspend the temperature elevation beyond the predetermined level is provided. A timer mechanism is attached for setting the microwave irradiation time arbitrarily.

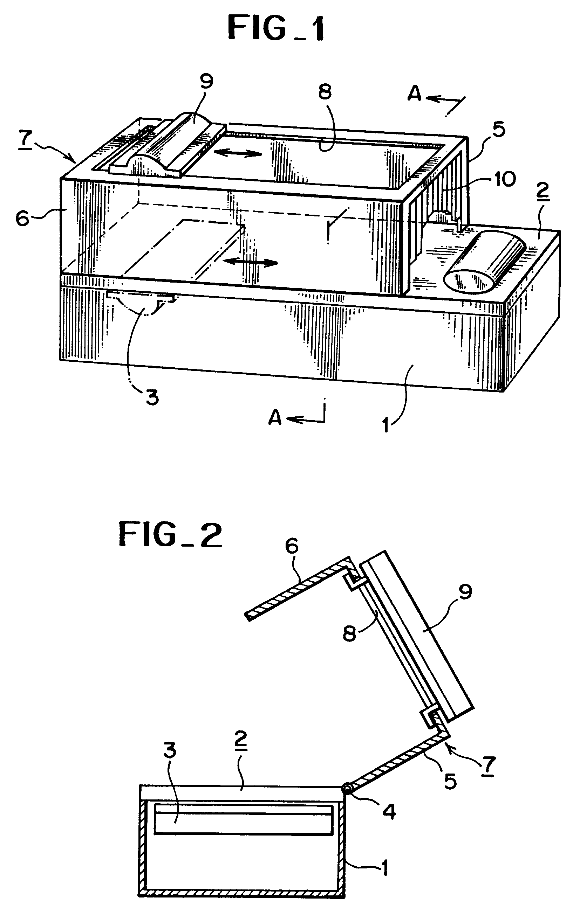

Reference will now be made to the preferred embodiment of device according to the present invention as shown in Figs. 1 and 2 for performing the above mentioned electronic treatment. In the figure, the reference numeral 2 denotes a bed, and 7 a protective frame of which cross section is shaped like an inverted U to be placed above the bed 2 along the longitudinal direction thereof.

The bed 2 is of a structure comprising a sheet made of a material which allows passing of microwaves, and is placed above a box shaped base 1 at an appropriate height above the floor, and incorporates a microwave irradiating means inside the base 1 below the bed 2 along the longitudinal direction of the bed 2 in a freely reciprocating fashion, the irradiation means 3 being inserted with a microwave irradiating tube 11 for irradiating the microwaves toward the bed 2.

The microwave irradiating means 3 can be moved reciprocally and freely along the longitudinal direction of the bed 2 by a known driving mechanism such as an endless chain and motor driving means 12, etc. driving said chain via a reduction gear over two parallel rails R,R mounted on the bed 2 along the longitudinal direction thereof. The range and speed of the reciprocal movement can be locally set by incorporating, for instance, a known microcomputer M.C., and a timer T.S. can further be incorporated within the device so that continuous irradiation for certain units of time such as 1 minute, 10 minutes, 30 minutes, 1 hour, 12 hours, 24 hours or more can be performed. It can also be structured so that irradiation can be stopped automatically after a predetermined period, or that irradiation can be resumed after the predetermined period of suspension.

The device is further connected to a temperature sensor 14 for detecting the surface temperature of the patient's body or the temperature of the lesion and be provided with an alarm means BZ or a means to automatically stop the temperature elevation beyond the prescribed temperature, so that the temperatures such as 40.5°C, 55.5°C and 60.5°C within the above mentioned temperature range of 37°C to 70°C can be stored in said microcomputer M.C to enable issuance of alarms when these temperatures are reached.

The alarm means BZ can be known voiced alarms such as a buzzer, or optical alarms such as lighting of pilot lamps colored red or yellow according to the temperature levels.

A means for automatically suspending the temperature elevation beyond the predetermined levels can be a switch coupled to above mentioned thermostat S.S.W.

It will be further advantageous if a suspension switch M.S.W. or an alarm means M.B.Z. which a patient can manually operate when he feels ill during a therapy session or when unusual circumstances such as too hot a temperature arises is provided.

The protective frame 7 has an opening 8 opening widely at the top thereof, and is mounted with a microwave irradiation means 9 along the longitudinal direction of the frame 7 in a freely reciprocating fashion striding over the left and right edges of said opening 8.

The microwave irradiation means 9 is identical in mechanism to the microwave irradiation means 3 which runs along the lower side of the bed 2, and its running (driving) mechanism is also identical. It is also provided with a microwave irradiating tube 15 along the opening 8 which irradiates microwaves toward the top surface of the bed 2 as it runs in reciprocating movement.

The intensity of microwaves being irradiated from the microwave irradiating devices 3, 9 is dependent on their running speed, but it should be set within the range not to cause burns in the patient. Therefore, it is more preferable to provide a sensor to automatically shut off the power when the intensity reaches a predetermined value which would pose dangers to the patient if exceeded and which has been selected in view of the intensity of microwave irradiated on the bed 2 and the running speed of the microwave irradiation devices 3, 9.

The microwave irradiation devices 3, 9 can be run in or out of synchronization with each other. They can be reciprocated over the whole length of the bed 2 or of the protective frame 7, or over the specified distance in order to concentrate irradiation at the lesion in a patient by using a control means such as a known microcomputer M.C., as mentioned above.

In the above mentioned case, a visible ray indicator means can be provided on the side of the microwave irradiation means 9, 3, the protective frame 7 or the base 1, to display the direction coincident with the direction of microwave irradiation, to thereby improve accuracy of the treatment and operational efficiency of the device.

At least the left and right side plates 5, 6 of the protective frame 7 having a cross section shaped like an inverted U are comprised of a shielding material which reflects and does not allow passage of microwaves, and the device is further provided with a screen 10 at the front of the protective frame 7 or toward the head of a patient lying on the bed 2, the screen made of a flexible and radiowave shielding material with vertical slits cut therein.

One side plate 5 of the protective frame 7 is mounted pivotally at one end of the bed 2 via a hinge mechanism 4, while a limit switch L.S. is provided at an appropriate place at the base of the side plate 6 at the opposite side (not shown). When the protective frame 7 is lowered over the bed 2 and locked, and when the base of the side plate 6 contacts the upper surface or the side surface of the bed 2, said limit switch L.S. is actuated to run the above mentioned microwave irradiation means 3, 9, or to close the circuit (to input) for microwave irradiation.

The above mentioned embodiment was explained in reference to a case where the microwave irradiation means 9, 3 were run over the bed 2 fixedly mounted on the base 1. If the space permits, the microwave irradiation means 9, 3 may be fixed, and the bed 2 provided with the protective frame 7 can be made movable in reciprocating manner along the longitudinal and horizontal direction.

In the construction as above mentioned, the patient is made to lie down on the bed 2 with the protective frame 7 opened over the side plate 6 via the hinge mechanism 4 manually or by a mechanical means as shown in Fig. 2, and after the patient lies down, the frame 7 is lowered over the bed 2. The patient is thus covered by the protective frame 7 except his head, and the head is shielded by the screen 10.

The lesion is confirmed by X-ray or other means with the patient thus positioned, and depending on the depth and size of the lesion, and the patient's conditions, the microcomputer M.C. issues commands based on the programmed details such as the range of reciprocity and speed of the microwave irradiation means 3,9, the intensity and duration of microwave irradiation and the heating temperature so that the microwave irradiation means 9 or the microwave irradiation means 9, 3 are energized to start microwave irradiation to the patient lying on the bed 2, and to cause the microwave irradiation means 9 or means 9, 3 to move in reciprocating movements at a constant speed over the whole length of the bed 2 along its longitudinal direction or over the prescribed length limited to the localized area.

Upon completion of the treatment, the protective frame 7 is opened upward and the patient is released from the bed 2.

The present invention achieves the following effects by the above mentioned construction.

As the treatment is designed by setting the temperature for heating the lesion by microwave irradiation arbitrarily within the range of 37 - 70°C, it is extremely effective for various forms of malignant tumors and other cellular or viral diseases.

The patient can be treated without taking off the clothes as the uniform heating can be given to as deep as the skeletal tissues in the patient's body.

Within the temperature range of 37 - 70°C, fine temperature adjustments for each 1°C up to 40°C and for each 0.5°C beyond 40°C are provided so that a speedier treatment with fine temperature control and fine adjustment for precisely destroying malignant tissues or viruses in the patient's body can be realized.

The device is provided with a switch M.S.W. to suspend the treatment by the patient or others by the provision of an alarm issuing means B.Z. when the temperature at the lesion reaches the predetermined levels of 40.5°C, 55.5°C, and 60.5°C within the above mentioned range of 37°C - 70°C so that the patient can arbitrarily set the heating means adapted to his individual needs, a means to automatically stop the temperature elevation beyond the predetermined temperature, a timer mechanism T.S. to set the microwave irradiation hours and speed arbitrarily, and an emergency means to tell the abnormality. This helps to assure the patient of his safety, and achieves the safe and efficient heating treatment using microwaves.

At least the right and left side plates of the protective frame are made of a radiowave shielding material, and a flexible screen made of a radiowave shielding material cut vertically along its vertical direction at the front of the protective frame as a need arises. These act to prevent leakage of microwaves during treatment and protect the nurses and attendants attending the patient.

At the top of the protective frame, or at the top of the protective frame and the bottom of the bed is provided with a microwave irradiation tube 11,15 toward the top of the bed in a freely reciprocating manner along the longitudinal direction of the bed to effect an efficient treatment at the optimum temperature at all times within the scope not to cause localized burns in the patient, and to apply the device to extensive electronic treatment by heating and destroying the tissues such as malignant tumors within the patient's body, etc.

The protective frame provided with the microwave irradiation device 9 is provided with a hinge mechanism between the lower end of one of the side plate and the bed to let the other side plate open upward above the bed, and helps the patient to simply and easily climb up and down the bed.

## Claims

1. An electronic treatment device for thermally treating at least one body portion of a patient supported by a bed (2), by emitting microwave energy toward the body portion of the patient to heat said body portion to a specific treatment temperature, said device comprising first microwave irradiation means (3) below said bed for emitting microwave energy towards said body portion, said irradiation means (3) being movable relative to said bed along the longitudinal direction of the bed, characterized by second microwave irradiation means (9) mounted above said bed (2) and being movable relative to said bed (2), said first and second microwave irradiation means (9,3) being adapted to heat the body portion of a patient to a temperature within the range of 37°C to 70°C; a protective frame (7) mounted on said bed (2) along the longitudinal direction thereof and having an inverted U-shape cross-section, with said second microwave irradiating means (9) being provided on the upper side of the protective frame (7) for relative reciprocation between the second microwave irradiating means and the bed; sensing means (14) for sensing the temperature in the body portion of a patient being irradiated; and warning means (BZ) responsive to said sensor means (14) for issuing a warning when the temperature in said body portion exceeds a predetermined value.

2. An electronic treatment device as claimed in Claim 1 comprising means to enable adjustment of the raising or lowering of said temperature in steps of 1°C if the temperature is below 40°C and in steps of 0.5°C if the temperature is above 40°C within said range of 37°C to 70°C.

3. An electronic treatment device as claimed in Claim 1 or Claim 2 comprising means enabling the heating temperature to be arbitrarily and finely adjusted by a patient to a selected level at which the patient feels comfortably warm within said temperature range of 37°C to 70°C.

4. An electronic treatment device as claimed in any one of Claims 1 to 3 further provided with a timer mechanism for arbitrarily setting the irradiation period of microwaves in a microwave irradiation treatment, within said temperature range of 37°C to 70°C.

5. An electronic treatment device as claimed in any one of Claims 1 - 4 wherein said protective frame (7) is hingedly mounted (4) on the bed (2) along a longitudinal side edge (5) thereof.

6. An electronic treatment device according to any one of Claims 1 - 5 further provided with a flexible screen (10) of microwave shielding material provided in an end wall of the frame (7) towards the head of a patient supported, in use, on said bed.

7. An electronic treatment device according to Claim 6 wherein said microwave shielding material is formed with a series of vertical slits therein.

## Patentansprüche

1. Elektronische Behandlungsvorrichtung zur thermischen Behandlung von wenigstens einem Körperabschnitt eines auf einem Bett (2) ruhenden Patienten durch Emittieren von Mikrowellen zu dem Körperabschnitt des Patienten, um den genannten Körperabschnitt auf eine spezifische Temperatur zu erwärmen, wobei die genannte Vorrichtung erste Mikrowellen abstrahlende Mittel (3) unterhalb des genannten Betts zur Emission von Mikrowellenenergie zu dem genannten Körperabschnitt aufweist, wobei die genannten abstrahlenden Mittel (3) relativ zu dem genannten Bett entlang der Längsrichtung des Betts bewegbar sind, gekennzeichnet durch zweite Mikrowellen abstrahlende Mittel (9, 3), die daran angepaßt sind, den Körperabschnitt des Patienten auf eine Temperatur innerhalb des Bereichs von 37 C bis 70 C zu erwärmen; einem am genannten Bett (2) entlang dessen Längsrichtung angeordneten Schutzrahmen (7) mit einem Querschnitt einer umgedrehten U-Form, wobei die genannten zweiten Mikrowellen abstrahlenden Mittel (9) an der oberen Seite des Schutzrahmens zur zwischen den zweiten Mikrowellen abstrahlenden Mitteln und dem Bett relativen Hin- und Herbewegung vorgesehen sind; Sensormittel (14) zum Erfassen der Temperatur in dem bestrahlten Körperabschnitt des Patienten; und auf die Sensormittel (14) reagierende Warnmittel (BZ) zur Ausgabe einer Warnung, wenn die Temperatur im genannten Körperabschnitt einen vorbestimmten Wert überschreitet.

2. Elektronische Behandlungsvorrichtung nach Anspruch 2, mit Mitteln für eine mögliche Anpassung der Erhöhung oder Verringerung der genannten Temperatur in Schritten von 1 C, wenn die Temperatur unter 40 C ist, und in Schritten von 0,5 C, wenn die Temperatur über 40 C ist, innerhalb des genannten Bereichs von 37 C bis 70 C.

3. Elektronische Behandlungsvorrichtung nach Anspruch 1 oder Anspruch 2, mit Mitteln zum Ermöglichen einer willkürlichen und Feinanpassung der Erwärmtemperatur durch einen Patienten auf einen ausgewählten Level, bei dem sich der Patient angenehm warm innerhalb des genannten Temperaturbereichs von 37 C bis 70 C fühlt.

4. Elektronische Behandlungsvorrichtung nach einem der Ansprüche 1 bis 3, ferner mit einer Zeitgebereinrichtung zum willkürlichen Einstellen der Zeitdauer der Bestrahlung mit Mikrowellen bei einer Mikrowellenbestrahlungsbehandlung innerhalb des genannten Temperaturbereichs von 37 C bis 70 C.

5. Elektronische Behandlungsvorrichtung nach einem der Ansprüche 1 bis 4, wobei der genannte Schutzrahmen (7) gelenkig am Bett (2) entlang einer Längsseitenkante (5) davon angeordnet ist.

6. Elektronische Behandlungsvorrichtung nach einem der Ansprüche 1 bis 5, ferner mit einer Blende (10) aus Mikrowellen abschirmendem Material, die in einer Stirnwand des Schutzrahmens (7) zum Kopf eines bei Benutzung auf dem genannten Bett ruhenden Patienten hin vorgesehen ist.

7. Elektronische Behandlungsvorrichtung nach Anspruch 6, wobei das Mikrowellen abschirmende Material mit einer Anzahl von vertikalen Schlitzen darin ausgebildet ist.

## Revendications

1. Dispositif de traitement électronique pour traiter thermiquement au moins une portion du corps d'un patient supporté par un lit (2), par émission d'une énergie micro-ondes en direction de la portion du corps du patient pour chauffer ladite portion du corps pour l'amener à une température de traitement spécifique, ledit dispositif comportant un premier moyen (3) d'émission de micro-ondes en dessous dudit lit pour émettre une énergie micro-ondes en direction de la dite portion du corps, ledit moyen d'émission (3) étant mobile par rapport audit lit, selon la direction longitudinale du lit, dispositif caractérisé par un second moyen (9) d'émission de micro-ondes monté au-dessus dudit lit (2) et mobile par rapport audit lit (2), le premier et le second moyens (9,3) d'émission des micro-ondes étant conçus pour chauffer la portion du corps d'un patient pour la porter à une température située sur la plage de 37°C à 70°C; par un cadre protecteur (7) monté sur ledit lit (2) selon sa direction longitudinale et présentant une section transversale en forme de U inversé, ledit second moyen (9) d'émission de micro-ondes étant prévu sur la face supérieure du cadre protecteur (7) pour effectuer un mouvement relatif de va-et-vient entre le second moyen d'émission des microondes et le lit; par des moyens détecteurs (14) pour détecter la température de la portion du corps d'un patient en train de recevoir les émissions; et par des moyens d'avertissement (B.Z.), sensibles auxdits moyens détecteurs 14) pour déclencher un avertissement lorsque la température dans ladite portion du corps dépasse une valeur prédéterminée.

2. Dispositif de traitement électronique salon la revendication 1, comportant des moyens pour permettre l'ajustement de la montée ou de la descente de ladite température par échelons de 1°C si la température est inférieure à 40°C et par échelon de 0,5°C si la température est supérieure à 40°C, sur ladite plage allant de 37°C à 70°C.

3. Dispositif de traitement électronique selon la revendication 1 ou la revendication 2, comportant des moyens permettant au patient d'ajuster la température de chauffage, à volonté et finement, à un niveau choisi auquel le patient ressent une chaleur confortable sur ladite plage de température de 37°C à 70°C.

4. Dispositif de traitement électronique selon l'une quelconque des revendications 1 à 3, comportant en outre un mécanisme temporisateur pour déterminer à volonté la période d'émission des micro-ondes dans un traitement d'émission de micro-ondes, sur ladite plage de température de 37°C à 70°C.

5. Dispositif de traitement électronique selon l'une quelconque des revendications 1 - 4 dans lequel ledit cadre protecteur (7) est monté par charnière (4) sur le lit (2) le long de l'un (5) de ses bords latéraux longitudinaux.

6. Dispositif de traitement électronique selon l'une quelconque des revendications 1 - 5 comportant en outre un écran flexible (10) de matériau réalisant un blindage à l'égard des micro-ondes prévu dans une paroi d'extrémité du cadre (7) en direction de la tête du patient supporté, en service, sur ledit lit.

7. Dispositif de traitement électronique selon la revendication 6, dans lequel, dans ledit matériau de blindage à l'égard des micro-ondes est prévue une série de fentes verticales.
